# EUROPEAN PATENT APPLICATION

(11) **EP 4 516 112 A1**
(43) Date of publication of application: **05.03.2025**
(21) Application number: 23306453.4
(22) Date of filing: 31.08.2023
(51) Int. Cl.: A23K 10/12, A01G 18/20, A23L 31/10, A01G 2/00, A61K 36/00, A23K 10/30, A23K 10/16

(54) **METHOD FOR PROCESSING SUGAR BEET BY-PRODUCT**

(71) Applicant: Green Spot Technologies, 31520 Ramonville-Saint-Agne (FR)
(72) Inventor: GRANUCCI, Ninna, 31400 Toulouse (FR); GALLO, Sandra, 31500 Toulouse (FR); SAINT UPERY GUNZEL, Elise, 31200 Toulouse (FR)
(74) Representative: Santarelli

(57) **Abstract**

The invention describes a method for processing a sugar beet by-product. This method makes it possible to reduce or eliminate the taste intensity and/or bitterness intensity and/or the smell intensity of a sugar beet by-product fermented by a fungus from the genus *Morchella.* Also described is a processed sugar beet by-product obtainable by the method according to the invention and compositions comprising it. This processed sugar beet by-product or composition is used as food or feed.

## Description

### Field of Invention

The present invention relates to a method for processing sugar beet by-product, resulting processed sugar beet by-product and uses thereof. More particularly, the method for processing involves a fermentation.

### Background to the invention

The sugar industry, by extracting sugar from sugar beet, generates by-products like sugar beet pulp. One ton of sugar beet yields on average 160 kg of sugar, 500 kg of sugar beet pulp, and 38 kg of molasses.

Sugar beet by-products, in particular sugar beet pulp, has an unpleasant smell, as well as an unpleasant taste and a strong bitterness. For these reasons, sugar beet by-products are usually used for landfield spreading, methanisation and in a lesser extend as animal feed for some specific animals that are tolerant to the unpleasant taste and smell. For example, sugar beet pulp can be used as feed for some ruminants or pigs.

However, even if tonnages are very high (more than three millions of tons per year), sugar beet by-products cannot be used as food and extensively used as feed because of their unpleasant taste and smell as well as their low nutritional profile.

Therefore, there is still a need to valorise this resource in food and feed: to do this, it is necessary to overcome the nauseating gustatory and olfactory barrier and to improve nutritional profile.

The inventors surprisingly found a method for processing sugar beet by-product allowing to obtain a product that does not present the above-mentioned drawbacks and can be used as food and feed.

The present invention relates to a method for processing a sugar beet by-product comprising the following steps:
a) preparing the sugar beet by-product by hydrating, drying and/or maintaining said sugar beet by-product to achieve a moisture level between 35 wt% and 95 wt%, the wt% being relative to the total weight of said sugar beet by-product;
b) sterilising the prepared sugar beet by-product thereby obtain a sterilised sugar beet by-product;
c) inoculating the sterilised sugar beet by-product with a fungus from the genus *Morchella* to obtain an inoculated substrate; and,
d) incubating the inoculated substrate to obtain a processed sugar beet by-product.

The method according to the invention makes it possible to reduce or eliminate the taste intensity and/or the smell intensity and/or the bitterness intensity of a sugar beet by-product. Preferably, the method according to the invention make it possible to reduce or eliminate the taste intensity, the smell intensity and the bitterness intensity of a sugar beet by-product.

It will be noted that in the context of the present application, and unless otherwise stipulated, the ranges of values indicated are understood to be inclusive.

By "reduce the intensity", it is intended to meant that the intensity is reduced of at least 15%, preferably at least 30%, more preferably at least 40% and sometimes 60% such as for the bitterness of the initial intensity.

In particular, reducing or eliminating an intensity is different from creating new flavours or aroma compounds that would mask the taste and/or the bitterness and/or the smell.

By "sugar beet", it is meant a plant namely *Beta vulgaris* belonging to the family of *Amaranthaceae.* However, sugar beet is different from the following denomination classes, which are based on designation made by the International Union for the Protection of New Varieties of Plants (UPOV): fodder beet group, spinach beet, mangold group or beetroot group (also commonly called beet, garden beet or common beet). In particular, sugar beet are used for the production of sucrose, and are bred for a high content of sugar, about 15 to 21 wt% (weight percent) relative to the weight of sugar beet, mainly soluble sugars (sucrose).

By "sugar beet by-product", it is meant a product that is produced incidental to the production process of sugar from sugar beet. The sugar industry generates several kinds of by-products, including in particular sugar beet pulp such as fresh sugar beet pulp, surpressed sugar beet pulp or dehydrated sugar beet pulp.

Preferably, in the method according to the invention, the sugar beet by-product is selected from the group consisting of fresh sugar beet pulp, surpressed sugar beet pulp, dehydrated sugar beet pulp and any mixtures thereof.

By "fresh sugar beet pulp", it is meant a sugar beet by-product obtained after juice extraction of the sugar beets. Usually, during sugar production, the sugar beets are first cleaned and shredded into small pieces named "cossettes" that are driven through a stream of hot water (usually between 60°C and 70°C) to extract the sugar contained in the beets. After this step, commonly named "diffusion", the liquid loaded in sugar continues the process of sugar refinery to extract and concentrate more the sugar. On the contrary, the fibrous material obtained after juice extraction of the beets is a sugar beet by-product and referred to, at this stage, as fresh sugar beet pulp. A fresh sugar beet pulp usually comprises more than 90% water, the percentage being on the total weigh of the fresh sugar beet pulp. Moreover, a sugar beet comprises between about 4-10% by weight of sugar, the percentage being on the total weigh of the dried sugar beet pulp.

By "surpressed sugar beet pulp", it is meant a sugar beet by-product obtained after a pressing step of the fresh sugar beet pulp. In particular, this pressing step removes water to reach around 70% moisture and to extract residual sugar to return this stream to the sugar refinery process. A surpressed sugar beet pulp usually comprises between about 3 and 7% by weight of sugar, the percentage being on the total weigh of the dried surpressed sugar beet pulp.

By "dehydrated sugar beet pulp", it is meant a sugar beet by-product obtained after a drying step of the fresh sugar beet pulp and/or the surpressed sugar beet pulp. A dehydrated sugar beet pulp usually comprises a moisture content around 10% by weigh relative to the total weigh of the product. The drying step usually allows to stabilize the molecules.

Whatever the sugar beet pulp (fresh, surpressed or dehydrated), the smell intensity, and the taste intensity and the bitterness intensity are very unpleasant enabling these sugar beet by-products to be only used for feed, land spreading or methanisation.

Advantageously, the sugar beet by-product can be used directly at the end of the sugar production process or the sugar beet by-product can be treated with a mechanical treatment selected from the group consisting of cutting, milling, grinding, pressing, shearing and chopping, prior to the preparation step a).

Preferably, the dehydrated sugar beet pulp is ground prior to the preparation step a).

The method according to the invention involves a fermentation step with a fungus from the genus *Morchella.*

Fungi from the genus *"Morchella"* are filamentous fungi from the Ascomycota division in the family Morchellaceae. According to the invention, *Morchella* can be a natural or engineered *Morchella,* and may be used under different form such as mycelium (free mycelium or pellet). Conventionally, a distinction is made between two types of morphology: non aggregated morphology called "free mycelium" in opposition to an aggregate morphology ("pellet").

The method according to the invention involves a fermentation step with a fungus from the genus *Morchella* selected from the species *Morchella anatolica, Morchella acerina, Morchella anthracina, Morchella anthracophila, Morchella rufobrunnea, Morchella Americana, Morchella castaneae, Morchella diminutiva, Morchella esculenta, Morchella dunensis, Morchella flu via lis, Morchella galilaea, Morchella palazonii, Morchella prava, Morchella sceptriformis, Morchella steppicola, Morchella ulmaria, Morchella vulgaris, Morchella angusticeps, Morchella arbutiphila, Morchella Australiana, Morchella brunnea, Morchella conifericola, Morchella deliciosa, Morchella disparilis, Morchella dunalii, Morchella elata, Morchella eohespera, Morchella eximia, Morchella eximioides, Morchella exuberans, Morchella feekensis, Morchella iberica, Morchella importuna, Morchella kakiicolor, Morchella laurentiana, Morchella magnispora, Morchella mediteterraneensis, Morchella populiphila, Morchella pulchella, Morchella punctipes, Morchella purpurascens, Morchella semilibera, Morchella septentrionalis, Morchella sextelata, Morchella snyderi, Morchella tomentosa, Morchella tridentina, Morchella anteridiformis, Morchella apicata, Morchella bicostata, Morchella conicopapyracea, Morchella crassipes, Morchella deqinensis, Morchella distans, Morchella guatemalensis, Morchella herediana, Morchella hetieri, Morchella hortensis, Morchella hotsonii, Morchella hungarica, Morchella inamoena, Morchella intermedia, Morchella meiliensis, Morchella miyabeana, Morchella neuwirthii, Morchella norvegiensis, Morchella patagonica, Morchella patula, Morchella pragensis, Morchella procera, Morchella pseudovulgaris, Morchella rielana, Morchella rigida, Morchella rigidoides, Morchella smithiana, Morchella sulcate, Morchella tasmanica, Morchella tatari, Morchella tibetica, Morchella umbrina, Morchella umbrinovelutipes or Morchella vaporaria.*

Preferably, the method according to the invention involves a fermentation step with a fungus from the species *Morchella hortensis, Morchella esculenta* or *Morchella brunnea.*

In order to prepare the substrate for fermentation, the sugar beet by-product is prepared such that it has a moisture content of between 35% and 95%, preferably between 45% and 80%, more preferably between 55% and 75%, and still more preferably about 70%, the percentage being expressed by weight relative to the total weigh of the sugar beet by-product.

These ranges of moisture allow good fermentation process in order to reduce or eliminate the taste intensity and/or the smell intensity and/or the bitterness intensity of the sugar beet by-product. They also allow improving the nutritional profile of the sugar beet by-product.

By "hydrating a sugar beet by-product", it is meant the addition of water to the sugar beet by-product if the sugar beet by-product has a lower moisture content than required.

Advantageously, the addition of water can be done in one or several times. For example, several amounts of water can be added to the sugar beet by-product over a period of time to ensure the water has been fully absorbed.

By "drying a sugar beet by-product", it is meant the removing of water from the sugar beet by-product if the sugar beet by-product has a higher moisture content than required.

In particular, the drying step of the sugar beet by-product can be performed by conventional means such as pressing or decanting.

Advantageously, the sugar beet by-product is prepared by a drying step followed by a rehydration step within the required ranges.

For sugar beet by-product having a moisture level within the required ranges, no hydration is required.

Advantageously, once the sugar beet by-product has been prepared, the prepared sugar beet by-product is sterilised. The sterilization step is advantageous because it makes it possible to prevent the multiplication of the molds and thus to stabilize the sugar-beet pulp. Without sterilisation, unwanted bacteria and yeasts could develop during fermentation and give an acidic, sour and unpleasant taste.

Advantageously, the sterilisation step is performed according to any standard sterilisation methods.

Advantageously, in step b) of the method, the sterilisation is performed by high temperature sterilisation or low temperature sterilisation, and/or high pressure sterilisation.

By "high temperature sterilisation" is typically defined as a sterilisation process that uses temperatures above 110°C, with optimal range being between 110°C and 170°C, and more advantageously between 160°C and 170°C, typically taking only a few minutes.

By "high pressure sterilisation" is typically defined as a sterilisation process that uses high pressures, typically between 0.8 and 3 bar, with more optimal range being between 1 and 2 bar, at temperatures between 105°C and 132°C, with more optimal range being between 115°C and 121°C, and typically taking between 1 and 30 minutes, with more optimal range being between 1 and 20 minutes.

By "low temperature sterilisation" is typically defined as a sterilisation process that uses temperatures generally lower than those used in high temperature sterilization, i.e., lower than 110°C, and typically takes several hours.

Advantageously, the sterilisation is performed by high temperature sterilisation and/or high pressure sterilisation. More advantageously, the sterilisation step is performed by high temperature sterilisation and high pressure sterilisation, and in particular at a temperature between 110°C and 130°C, during 1 to 20 min and at a pressure comprised between 0.5 and 2 bar.

Advantageously, the method comprises a further step of adding a functional additive to the sugar beet by-product prior to inoculation.

In particular, this functional additive can be helpful in the growth of the fungus from the genus *Morchella* during the fermentation. In particular, this functional additive can be helpful in the growth of the fungus from the genus *Morchella* to fully colonize the sugar beet by-product until the colonized sugar beet by-product forms a block held by the mycelium spreads in the sugar beet by-product.

Advantageously, the functional additive is added prior to the sterilization step. Alternatively, or in addition, it can be added after the sterilization step.

Advantageously, it is selected from the group consisting of mineral salts, vitamins, organic acids such as citric acid and any mixtures thereof.

Preferably, the functional additive is a food or feed grade nitrogen source.

Advantageously, the food or feed grade nitrogen source is selected from the group consisting of ammonium sulphate, glutamic acid, yeast extract, peptone and di-ammonium phosphate (DAP), and any mixtures thereof.

Advantageously, the food or feed grade nitrogen source is added in an amount comprised between 0.1 wt.% and 25 wt.%, preferably between 0.5 wt.% and 10 wt% relative to the total weight of the prepared sugar beet by-product. More advantageously, the food grade nitrogen is added to the prepared sugar beet by-product at about 1 % by weight relative to the total weight of the substrate.

By "about [a value of] X", it is intended to mean more or less 10% of the value of X.

Advantageously, following to the step b), the sterilised sugar beet by-product is cooled to room temperature.

In step c), the sterilised sugar beet by-product is inoculated with a fungus from the genus *Morchella.* The fermentation process relies on the use of an active *Morchella* as inoculant to be used to colonise the sterilised sugar beet by-product as a substrate.

Advantageously, the fungus from the genus *Morchella* is added to the sterilised sugar beet by-product under the form of mycelium. In that case, 0.5 wt% to 30 wt%, preferably 1 wt% to 10 wt% of mycelium of the fungus from the genus *Morchella* is added, the wt% being relative to total weight of the sterilised sugar beet by-product.

In step d), the inoculated sugar beet by-product is incubated.

By "incubating", it is intended to mean a step during which the microorganism (i.e. the fungus from the genus *Morchella)* grows, and during which fermentation takes place.

In the method according to the invention, the fermentation is a solid-state fermentation.

By "solid-state fermentation", it is meant a fermentation using solid substrate, contrary to liquid or submerged fermentation, wherein the microorganisms grow in liquid medium, with high content of free water. The required liquid (water) content in solid state fermentation is absorbed by the solid substrate. Thus, during solid-state fermentation, the growth of the selected microorganism(s) occurs on solid materials.

Advantageously, in step d), the inoculated sterilised sugar beet by-product is incubated for a period of 1 to 32 days.

More advantageously, the incubation (step d)) is performed for a period of 5 to 32 days, still more preferably for a period of 10 to 30 days.

Advantageously, the incubation is performed at a temperature comprised between 5°C and 50°C, more preferably between 12°C and 35°C, still more preferably between 18°C and 30°C.

Humidity and air flow conditions during incubation can advantageously be controlled to obtain optimal growth of the fungus from the genus *Morchella.*

Advantageously, humidity in the incubation space should be kept to a maximum to facilitate fungal growth, preferably between 50-100% humidity.

Advantageously, air flow around the substrate during incubation should also be managed to prevent build-up of either excessive oxygen or carbon dioxide.

Advantageously, the CO₂ levels are held between 1% and 30%, preferably between 1% and 10%.

Advantageously, the O₂ levels are held between 1% and 30%, preferably between 1% and 20%.

Advantageously, the inoculated sterilised sugar beet by-product is incubated in vessels that can be in glass, plastic or stainless steel.

In the method according to the invention, the processed sugar beet by-product can be subjected to at least one further step selected from the group consisting of rehydrating, thermal treatment, drying, milling, granulating and any combinations thereof.

Preferably, the processed sugar beet by-product is subjected to a thermal treatment, which allows to inactivate the myceliums.

Advantageously, the thermal treatment is performed at a temperature comprised between 75°C and 121°C, more advantageously between 85°C and 116°C.

Advantageously, the thermal treatment is performed during a time duration comprised between 1 minutes and 30 minutes, more advantageously between 1 minutes and 12 minutes.

Advantageously, the processed sugar beet by-product is subjected to a drying step. Drying has the effect of stabilizing the processed sugar beet by-product.

Advantageously, the step of drying the processed sugar beet by-product is performed at a temperature of between 40°C and 250°C, more advantageously, between 50°C and 150C:

In one embodiment, the drying temperature is maintained below 75°C, such as 60°C. In the present embodiment, the step of drying is performed during a time duration between 10 min and 24 hours, preferably between 1h and 15 hours, most preferably between 2 hours and 10 hours.

In another embodiment, the drying temperature is maintained above 100°C, such as 120°C. In this another embodiment, the step of drying is performed during a time duration between 30 seconds and 10 minutes, preferably between 1 minute and 8 minutes, most preferably between 2 minutes and 6 minutes.

The combination of the drying temperature and the time duration presented in the two above embodiments, advantageously preserves the nutritional value of the composition. In particular, it prevents the breakdown of antioxidants, vitamins or proteins, etc.

Advantageously, the processed sugar beet by-product is dried until it becomes millable, typically when the moisture content is 10% by weight or less. At moisture content above 10% by weight, microorganisms may grow, affecting the taste, aroma and shelf life of the product.

Advantageously, the drying step is carried out via any techniques known to the skilled person, such as by drying at the cold or hot air, by under vacuum or by lyophilisation.

More advantageously, the step of drying occurs under vacuum conditions.

By "vacuum conditions" it intended to means preferably conditions wherein the pressure inside a sealed container or chamber is significantly reduced below the ambient atmospheric pressure.

It is advantageous to perform the drying steps under vacuum conditions using a freeze drying process. Furthermore, an optimal freeze drying process involves a primary drying phase under low pressure, such as below 200 µbar, at a temperature below -10°C, such as a primary drying at a pressure of 80 µbar at -20°C, followed by secondary drying, at a temperature above 0°C and at a pressure below the pressure of the primary drying, such as at a maximum temperature of 40°C and a pressure of 20 µbar. Usually, with the entire cycle comprising primary and secondary drying lasts about 24 hours.

Under vacuum conditions, the processed sugar beet by-product has a lighter or whiter final colour.

Advantageously, the processed sugar beet by-product is subjected, in the following order, to a thermal treatment, a drying and a milling.

Advantageously, the milling step is performed by using standard milling techniques.

Still more advantageously, the processed sugar beet by-product is subjected, in the following order, to a rehydration, a thermal treatment, a drying and a milling.

The invention further relates to the use of the method according to the invention, for reducing or eliminating the taste intensity and/or bitterness intensity and/or the smell intensity of a sugar beet by-product.

Thus, the method according to the invention can be a:
- method for reducing or eliminating the taste intensity of a sugar beet by-product and/or
- method for reducing or eliminating bitterness intensity of a sugar beet by-product and/or
- method reducing or eliminating the smell intensity of a sugar beet by-product.

The invention also provides a processed sugar beet by-product obtainable by the method according to the invention.

By "processed sugar beet by-product", it is meant a sugar beet by-product having the following features:
∘ it is fermented, *i.e.*, it has undergone a fermentation;
∘ it is edible, *i.e.*, it is suitable to be used as food by humans orfeed by the animal;
∘ it is acceptable in terms of palatability, *i.e.*, the intensity of the taste and/or the intensity of the bitterness and/or the intensity of the smell is reduced or eliminated.

The invention also concerns a composition comprising or consisting of sugar beet by-product fermented by a fungus from the genus *Morchella.*

Advantageously, the composition comprising or consisting of sugar beet by-product fermented by a fungus from the genus *Morchella,* contains at least 10% by weight of protein, preferably at least 13% by weight, more preferably at least 15% by weight, as a percentage of the total weight of the dry matter of the fermented sugar beet by-product.

Advantageously, the composition comprising or consisting of sugar beet by-product fermented by a fungus from the genus *Morchella,* preferably contains less than 4% by weight of sugar, more preferably less than 3% by weight as a percentage of the total weight of the dry matter of the fermented sugar beet by-product.

Finally, the invention relates to the use of the processed sugar beet by-product according to the invention, as food or feed.

According to a further aspect of the invention, there is provided the use of a processed sugar beet by-product obtained according to the method of the invention having an improved nutritional content.

The invention is particularly adapted to the production of a fermented sugar beet by-product for the food or feed industry.

According to a further aspect of the invention, there is provided the use of a processed sugar beet by-product obtained according to the method of the invention to replace entirely or partially the traditional flour, such as wheat, used for the manufacture of food or feed.

The food may be, for example, brownie, salty biscuit such as cracker or meat analogue.

The feed may be, for example, wet dog food.

### Brief description of the Drawings

Other features and advantages of the invention will become clear from the following examples, given by way of illustration, with reference to the figures, which represent respectively:

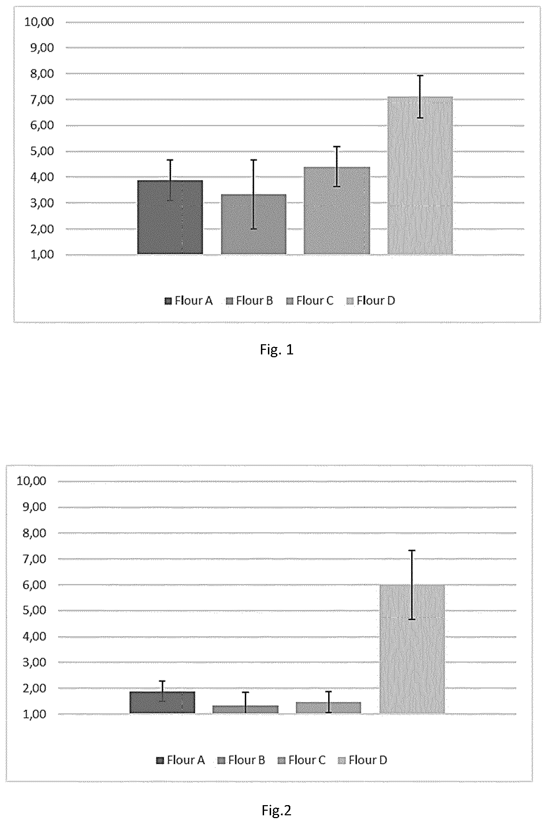

- Figure 1 is a diagram comparing the taste intensity (Mean grade given by the panel in taste intensity) of a sugar beet by-products processed by the method according to the invention (Flour A: Sugar beet by-product fermented with *Morchella hortensis*) or (Flour B: Sugar beet by-product fermented with *Morchella esculenta*) or (Flour C: Sugar beet by-product fermented with *Morchella brunnea*) with Flour D, Unfermented sugar beet by-product.
- Figure 2 is a diagram comparing the bitterness intensity (Mean grade given by the panel in taste intensity) of a sugar beet by-products processed by the method according to the invention (Flour A: Sugar beet by-product fermented with *Morchella hortensis*) or (Flour B: Sugar beet by-product fermented with *Morchella esculenta*) or (Flour C: Sugar beet by-product fermented with a fungus from the specie *Morchella brunnea*) with Flour D, Unfermented sugar beet by-product.

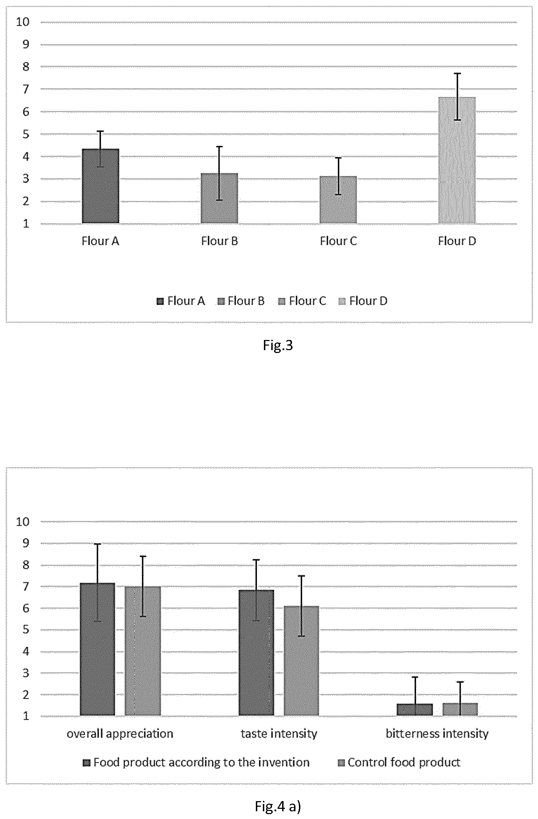

- Figure 3 is a diagram comparing the smell intensity (Mean grade given by the panel in taste intensity) of a sugar beet by-product processed by the method according to the invention (Flour A: Sugar beet by-product fermented with *Morchella hortensis*) with Flour D, Unfermented sugar beet by-product.

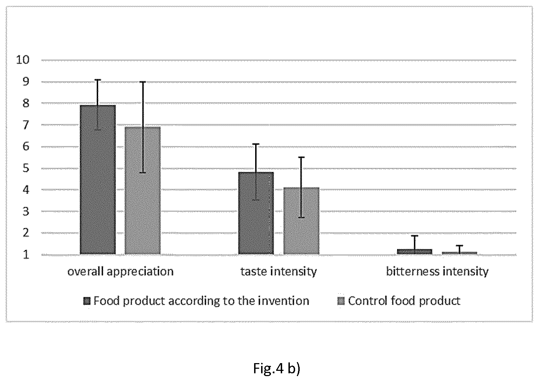

- Figure 4 is a diagram comparing the overall appreciation, as well as the taste intensity and bitterness intensity of different products; i.e. brownies (Figure 4 a)) and crackers (Figure 4 b)) comprising flour made from a sugar beet by-product processed according to the invention and fermented by *Morchella hortensis* (food product according to the invention) or comprising a traditional flour (control food product).

### Example 1: Method for obtaining a processed sugar beet by-product according to the invention

**Microorganism:** a fungus from the species *Morchella hortensis, Morchella esculenta, or Morchella brunnea* was used.

**Sugar beet by-product:** dehydrated sugar beet pulp was obtained from a sugar refinery based in the North of France. This sugar beet by-product was stored at 20°C until used.

**Culture medium:** sugar beet by-product was used for the solid-state fermentation. 500g of sugar beet by-product was rehydrated to obtain a substrate with a moisture content of 70% by weight relative to the total weight of the substrate. Then, the substrate was mixed with a 1% by weight of diammonium phosphate (nitrogen source) relative to the total wet weight of the substrate, into a container with caps, suitable for autoclave. The substrate is sterilized by autoclaving at 121°C for 20 minutes and at 1 bar.

**Inoculum:** myceliums of *Morchella hortensis, Morchella esculenta or Morchella brunnea grown in* a *Potato Dextrose Agar plate.*

***Culture conditions.*** The substrate is inoculated with 1% by weight of mycelium of *Morchella hortensis, Morchella esculenta or Morchella brunnea,* the percentage being expressed on the total weight of the substrate. The inoculated substrate is then incubated at 25°C for 28 days. During this step, the fermentation occurs. After fermentation, a thermal treatment with autoclave at 121°C for 5 min and at 1 bar, is applied on the substrate fermented to inactivate the myceliums. The substrate is removed from the container and dried until a moisture lower than 10%, and milled to obtained Flours A, B or C, respectively.

### Example 2: Control

***Sugar beet by-product:*** dehydrated sugar beet by-product was obtained from the same sugar refinery as Examples 1 and 2. This sugar beet by-product was stored at 20°C until used.

***Preparation:*** 500g of sugar beet by-product are milled to obtain Flour D.

### Example 3: Determination of taste, smell and bitterness intensities of the processed sugar beet by-product according to the invention, compared to a control (unprocessed sugar beet by-product)

### 1. Materiel and methods

Tasting, smelling and bitterness tests were carried out on an internal panel of 20 persons, on the flours obtained. The taste test consists in evaluating the taste and bitterness intensities, whereas the smelling test consists in evaluating the smell intensity.

Concerning the taste test, Flours A, B, C (as described in example 1), and D (control, non-fermented sugar beet pulp, as described in example 2) were rehydrated at a rate of 1g in 20 mL of water. The taste test was carried out in the 6 hours following rehydration of flours.

This rehydration is not implemented for the smell test: the flours were used directly dry.

Then, each flour was presented in a small pot, foiled with aluminium for the test to be blind.

For each parameter (taste intensity, bitterness intensity, smell intensity), a score is fixed. For each parameter, the score grades between 1 (low intensity) and 10 (high intensity), then the results obtained are compared to those obtained by the control.

### 2. Results

The results from Fig. 1 show that the taste intensity of Flours A, B or C according to the invention is significantly reduced compared to the control (Flour D).

The results from Fig. 2 show that the bitterness intensity of Flours A, B or C according to the invention is significantly reduced compared to the control (Flour D).

The results from Fig. 3 show that the smell intensity of Flours A, B or C according to the invention is significantly reduced compared to the control (Flour D).

### Example 4: Nutritional profile of the processed sugar beet by-product according to the invention

### 1. Materiel and methods

**Processed sugar beet by-product (Flours A, B, or C):** the processed sugar beet by-product are obtained according to example 1.

**Control sugar beet by-product (Flour D):** sugar beet by-product is obtained according to example 2.

**Analytical methods:** The analysis of the flours was conducted by an external provider, using established techniques, and in particular:
- the humidity in the flours was measured by drying and gravimetric analysis;
- the dry matter in the flours was calculated by the following equation: 100 - Humidity (%);
- the sugar content in the flours was analyzed by ion chromatography with pulsed amperometric detection (CI-PAD), after solvent extraction;
- the protein content in the flours was measured according to the Dumas method, also known as the combustion method;
- the metabolizable carbohydrate content in the flours was calculated by the following equation: metabolizable carbohydrate = 100 - (moisture + ash + lipid content + protein content + total dietary fibers) * 100%;
- the lipid content in the flours was measured by hydrolysis and solvent extraction, and gravimetric analysis;
- the ash content in the flours was measured by dry mineralization and gravimetric analysis; and,
- the total dietary fibers in the flours was measured by enzymatic digestion and gravimetric analysis (internal method adapted from AOAC 991.43).

### 2. Compositions of flours

The compositions of flours A, B, C and D are presented in Table 1 below.

**Table 1**

| | Flour A | Flour B | Flour C | Flour D |
|---|---|---|---|---|
| Humidity (%) | 5.8% | 6.3% | 7.6% | 7% |
| Dry matter (%)* | 94.2% | 93.7% | 92.4% | 93% |
| Sugar (%)** | 1.91% | 2.81% | 1.34% | 5% |
| Proteins (%) ** | 21.8% | 15.5% | 18.3% | 8% |
| Metabolizable carbohydrate (including sugar) (%)** | 18.3% | 22.8% | 13.9% | 9% |
| Lipid (%)** | 2% | 1% | 1.7% | 1.5% |
| ash (%)** | 11.4% | 8.8% | 10.4% | 8.5% |
| Total dietary fibers (%)** | 40.7% | 45.6% | 48.1% | 66% |

| | | | | |
|---|---|---|---|---|
| *% *dry matter relative to the total weight of the flour* **% *in dry weight relative to the total weight of the dry matter* | | | | |

The results in Table 1 illustrates the variations in composition between a flour made from non-fermented sugar beet (Flour D), sugar beet fermented by *Morchella hortensis* (Flour A), sugar beet fermented by *Morchella esculenta* (Flour B) and sugar beet fermented by *Morchella brunnea* (Flour C). It can be seen that the flour fermented by *Morchella hortensis* (Flour A) has more proteins and less sugar than the flour made from non-fermented sugar beet (Flour D).

### Example 5: Food product comprising the processed sugar beet by-product according to the invention

### 1. Material and Methods

The flour made from fermented sugar beet by-product with *Morchella hortensis* described in Example 1 is used for the manufacture of brownies and crackers.

Preparation of the brownies: [the amounts of the ingredients are adequately introduced to obtain the compositions described in table 2, hereafterl
- preheat the oven at 160°C;
- melt the chocolate in a water bath. When it is homogeneous, add oil and margarine;
- in the kenwood bowl, stir together egg, sugar and glucose fructose syrup using the whip for 2 minutes at speed 5;
- add the blend chocolate, oil and margarine, mix for 2 minutes at speed 5;
- premix the other powders together: flour(s), baking powder, cocoa powder, mono and diglycerides of fatty acids and salt;
- add the premix to the rest and the nuts, mix using the K-beater for 30 s at speed 2, then 1 min 30 s at speed 4;
- put baking paper in a mould; and,
- pour the mix in the mould and cook in the oven for 45 minutes.

Preparation of the crackers: [the amounts of the ingredients are adequately introduced to obtain the compositions described in table 2, hereafter)
- mix all the dry ingredients in the Kenwood bowl with the K beater;
- add the wet ingredients and mix until obtaining an homogeneous ball of dough during 3 minutes at speed 2;
- cover the ball with plastic film and put to the fridge during 30 min;
- preheat the oven at 160°C;
- spread the dough with the rolling meal until the thickness "3";
- cut the dough with a punch (diameter 4,5cm); and,
- cook in the oven for 15 minutes on an oven plate with baking paper

Composition of these food products are described in the following Table 2:

**Table 2**

| | Food product according to the invention | | Control food product | |
|---|---|---|---|---|
| | Brownie | Crackers | Brownie | Crackers |
| Flour according to the invention (%)* | 3.00 | 3.00 | 0 | 0 |
| Wheat flour (%) * | 7.84 | 55.11 | 10.84 | 58.11 |
| Sunflower oil (%)* | 15.22 | 14.40 | 15.22 | 14.40 |
| Egg (%)* | 20.12 | 14.40 | 20.12 | 14.40 |
| White sugar (%)* | 18.87 | 2.88 | 18.87 | 2.88 |
| Baking powder (%)* | 0.29 | 0.58 | 0.29 | 0.58 |
| Salt (%)* | 0.08 | 2.00 | 0.08 | 2.00 |
| Water (%)* | | 7.63 | | 7.63 |
| Dark chocolate (%)* | 18.70 | 0 | 18.70 | 0 |
| Cocoa powder without sugar (%)* | 1.55 | 0 | 1.55 | 0 |
| Margarine 70% of fat (%)* | 5.07 | 0 | 5.07 | 0 |
| Glucose fructose syrup (%)* | 6.29 | 0 | 6.29 | 0 |
| Mono and diglycerides of fatty acids (%)* | 0.07 | 0 | 0.07 | 0 |
| Nuts pieces (%)* | 2.90 | 0 | 2.90 | 0 |

| | | | | |
|---|---|---|---|---|
| * The percentage is expressed by weight relative to the total weight of the composition | | | | |

### 2. Results

The analysis methods are identical to those described in Example 4.

The results from Fig. 4 show that food products according to the invention were judged as good as the control food products.

The results from Fig. 4.a) show that the overall appreciation, the taste intensity and the bitterness intensity of the preparation of brownies, wherein Flour A according to the invention is incorporated, is similar to the control food product.

The results from Fig. 4.b) show that the overall appreciation, the taste intensity and the bitterness intensity of the preparation of crackers, wherein flour A according to the invention is incorporated, is similar to the control food product.

Moreover, other kind of food products comprising a processed sugar beet pulp according to the invention were formulated e.g., meat analogue. The smell was pleasant and the taste was judged acceptable. The results are not shown.

## Claims

1. A method for processing a sugar beet by-product comprising the following steps:
a) preparing the sugar beet by-product by hydrating, drying and/or maintaining said sugar beet by-product to achieve a moisture level between 35 wt% and 95 wt%, the wt% being relative to the total weight of said sugar beet by-product;
b) sterilising the prepared sugar beet by-product thereby obtain a sterilised sugar beet by-product;
c) inoculating the sterilised sugar beet by-product with a fungus from the genus *Morchella* to obtain an inoculated substrate; and,
d) incubating the inoculated substrate to obtain a processed sugar beet by-product.

2. The method according to claim 1, wherein the sugar beet by-product is selected from the group consisting of fresh sugar beet pulp, surpressed sugar beet pulp, dehydrated sugar beet pulp and any mixtures thereof.

3. The method according to claim 1 or 2, wherein in step b), the sterilisation is performed by high temperature sterilisation, high pressure sterilisation and/or low temperature sterilisation.

4. The method according to any of claims 1 to 3, wherein the method comprises a further step of adding a functional additive to the sugar beet by-product prior to inoculation.

5. The method according to claim 4, wherein the functional additive is a food orfeed grade nitrogen source.

6. The method according to any of claims 1 to 5, wherein in step d), the inoculated sterilised sugar beet by-product is incubated for a period of 1 to 32 days.

7. The method according to any of claims 1 to 6, wherein the processed sugar beet by-product is subjected to at least one further step selected from the group consisting of rehydrating, thermal treatment, drying, milling, granulating and any combinations thereof.

8. The method according to claim 7, wherein the step of drying the processed sugar beet by-product is performed at a temperature of between 40°C and 250°C.

9. The method according to claim 7 or 8, wherein the step of drying occurs under vacuum conditions.

10. Use of the method according to any of claims 1 to 9, for reducing or eliminating the taste intensity and/or bitterness intensity and/or the smell intensity of a sugar beet by-product.

11. A processed sugar beet by-product obtainable by the method according to any of claims 1 to 9.

12. A composition comprising or consisting of sugar beet by-product fermented by a fungus from the genus *Morchella.*

13. The composition of claim 12, comprising at least 10% by weight of proteins, as a percentage of the total weight of the dry matter of the fermented sugar beet by-product.

14. Use of the processed sugar beet by-product according to claim 11 or of the composition according to claim 12 or 13, as food or feed.
